(19) Europäisches Patentamt
European Patent Office
Office européen des brevets

(11) Numéro de publication: **0 576 420 B1**

(12) **FASCICULE DE BREVET EUROPEEN**

(45) Date de publication de fascicule du brevet: **31.05.95**

(51) Int. Cl.$^6$: **C07H 15/18**, A61K 7/48, A61K 31/70

(21) Numéro de dépôt: **91907075.5**

(22) Date de dépôt: **21.03.91**

(86) Numéro de dépôt internationale :
**PCT/FR91/00229**

(87) Numéro de publication internationale :
**WO 92/16544 (01.10.92 92/25)**

# (54) NOUVEAU DERIVE DE L'ACIDE CAFEIOUE, L'ORAPOSIDE, COMPOSITION COSMETIOUE OU PHARMACEUTIOUE, NOTAMMENT DERMATOLOGIOUE LE CONTENANT.

(43) Date de publication de la demande:
**05.01.94 Bulletin 94/01**

(45) Mention de la délivrance du brevet:
**31.05.95 Bulletin 95/22**

(84) Etats contractants désignés:
**AT BE CH DE DK ES FR GB GR IT LI LU NL SE**

(56) Documents cités:
**FR-A- 2 302 745**
**FR-A- 2 314 725**
**FR-A- 2 652 086**

**Chemcial Abstracts, Vol. 106, No. 19, 11 May 1987 (Columbus, Ohio, US), see page 574, abstract 155103s**

(73) Titulaire: **PARFUMS CHRISTIAN DIOR**
**33, avenue Hoche**
**F-75008 Paris (FR)**

(72) Inventeur: **ANDARY, Claude**
**642, avenue de la Justice**
**F-34090 Montpellier Cédex (FR)**
Inventeur: **ANDRE, Patrice**
**9, rue Charles**
**F-45170 Neuvilles-aux-Bois (FR)**

(74) Mandataire: **Portal, Gérard et al**
**Cabinet Beau de Loménie**
**158, rue de l'Université**
**F-75340 Paris Cédex 07 (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 576 420 B1

Chemical Abstracts, Vol. 107, No. 6, 10 August 1987, (Columbus, Ohio, US) S. Kitagawa et al.: "Studies on the Chinese crude drug "Forsythiae fructus. VIII. On isolation of phenylpropanoid glyosides from fruits of forsythia koreana and their antibacterial activity", see page 407, abstract 46141c, & Yakugaku Zasshi 1987, 107(4), 274-8

Chemical Abstracts, Vol. 107, No. 7, 17 August 1987 (Columbus, Ohio, US) Y. Limura et al.: "Effects of caffeoylglycosides on arachidonate metabolism in leukocytes", see page 37, abstract 51592, & Planta Med. 1987, 53(2), 148-53

Chemical Abstracts, Vol. 114, No. 10, 11 March 1991, see page 423, abstract 88423w, & JP.A. 2255607 (PIAS CO., LTD) 16 October 1990

## Description

La présente invention concerne essentiellement un nouveau dérivé de l'acide caféique, l'oraposide, une composition cosmétique ou pharmaceutique, notamment dermatologique le contenant.

Plus particulièrement, le nouveau dérivé de l'invention, l'oraposide, est un dérivé de l'acide caféique extrait de végétaux de la famille des Orobanchaceae. L'invention couvre également des dérivés particuliers de l'oraposide, notamment des dérivés acylés, ainsi que les extraits de plante en contenant, notamment les extraits d'Orobanche rapum genistae.

Des études préliminaires avaient déjà révélé la présence de dérivés de l'acide caféique dans les extraits d'Orobanchacées, plantes phanérogames parasites sans chlorophylle (Andary et al. 1980, Il Farmaco, 1, 1-30 ; Bridel et Charaux, 1924, C.R. Acad. Sci. 178, 1839) mais leur structure n'était pas clairement établie.

L'intérêt pharmacologique de l'acide caféique (ou acide 3,4-dihydroxycinnamique) et de plusieurs dérivés naturels de celui-ci a déjà fait l'objet de nombreuses études. Ainsi Kimura et al. (Planta Medica, 1984, 473-477) ont étudié l'effet de divers tanins et en particulier de dérivés de l'acide caféique et caféoylquinique extraits de plantes de l'espèce Artemisia et ont montré leur effet inhibiteur sur la peroxydation des lipides dans les mitochondries et les microsomes de foie de rat. L'action inhibitrice de ces molécules sur les lipoxygénases participant au métabolisme des leucotriènes et de l'acide arachidonique et leur utilisation potentielle dans le traitement d'affections inflammatoires comme l'asthme ont aussi été publiées par Kimura et Okuda (J. of natural products, 1987, 50, 392-399).

Le document FR-A-2,302,745 décrit des esters heterosidiques de l'acide caféique et des médicaments les contenant pour le traitement de la maladie de Parkinson (page 4, lignes 11 à 16 et revendications 1 à 14).

Le document CA 107: 46141C décrit l'isolement de phenyl propanoide glycoside à partir de fruits de Forsythia Koreana et leurs activités antibactériennes contre le Staphylococcus aureus.

L'activité de différents dérivés caféiques étant multiple et variable en fonction de leur structure, il est intéressant de rechercher de nouvelles molécules de cette famille, dans les plantes médicinales ou autres, et d'en chercher de nouvelles activités pharmacologiques ou des performances supérieures.

Ainsi, il a été obtenu, à partir d'extraits de plantes de la famille des Orobanchaceae qui parasitent divers genêts (Cytisus scoparius et purgans) ainsi que des plantes alimentaires comme les lentilles, les fèves, les solanées (tomates, aubergines...), un nouveau composé présentant des propriétés particulièrement intéressantes. L'Orobanche est une plante vivace représentée par une tige fleurie en épi, la plante entière peut atteindre une longueur de 20 à 100 cm avec des écailles le long de la tige qui se terminent à la base par un bulbe recouvert également d'écailles. Toute la plante est riche en dérivés esters hétérosidiques caféiques. Le bulbe, l'organe fixé sur les racines de la plante-hôte par des crampons ayant la forme de petites racines, est l'organe le plus riche en ces esters hétérosidiques caféiques.

La substance extraite de l'Orobanche rapum genistae a été obtenue à l'état pur et cristallisé, par exemple par macération alcoolique d'un extrait de plante ou de cellules de plante et soumise aux diverses analyses classiques permettant d'en établir la configuration moléculaire et la structure (séparations chromatographiques, détermination du point de fusion, études des spectres RMN, de masse, aux rayons X, IR et UV et diverses analyses biochimiques). La formule qui a été déduite de ces analyses est la suivante : 4-caféate de 3-0-($\alpha$-L-rhamnopyranosyl)-[2-(3,4-dihydroxyphényl)-1,2-éthylidène]-$\beta$-D-glucopyranoside.

Cette substance, que l'on a proposé d'appeler "oraposide", est représentée par la formule (I) ci-après, dans laquelle R est un atome d'hydrogène.

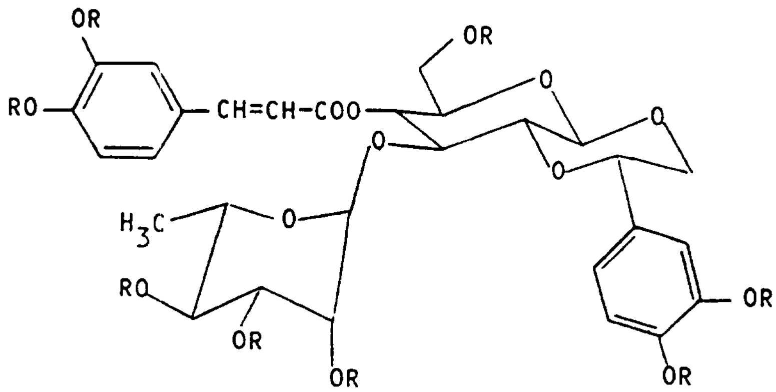

(I)

La présente invention concerne également les dérivés de formule I dans laquelle un ou plusieurs R représentent indépendamment un atome d'hydrogène, un alkyle inférieur en $C_1$-$C_5$, en particulier méthyle, ou un groupe acyle, en particulier en $C_1$-$C_6$, notamment acétyle.

Selon une autre caractéristique préférée, seuls les groupes OR portés par les cycles phényle sont alkylés, notamment méthylés.

De préférence, seuls les groupes OR portés par les cycles glucopyranosyle ou rhamnopyranosyle sont acylés, notamment acétylés.

En particulier, l'oraposide ou ses dérivés sont extraits à partir d'une plante de la famille des Orobanchacées, en particulier Orobanche rapum genistae.

On peut obtenir de tels dérivés par des méthodes classiques bien connues de l'homme de l'art.

Notamment pour préparer des dérivés peracétylés de l'oraposide, on peut opérer suivant une méthode simple qui consiste en ce que les cristaux d'oraposide sont dissous dans un volume d'anhydride acétique auquel on ajoute un volume de pyridine. On laisse une nuit entiére à la température ambiante. Le lendemain, on filtre, on ressolubilise le dérivé de l'invention et on lyophilise. On obtient ainsi le dérivé peracétylé de l'oraposide, c'est-à-dire que tous les groupes OR portés par les cycles glucopyranosyle ou rhamnopyranosyle sont ici acétylés.

Il a maintenant été découvert, de façon inattendue, que l'oraposide et ses dérivés précités possédaient de nombreux effets chimiques et biochimiques tels que piégeur de radicaux libres, antioxydant - prévenant notamment la peroxydation des lipides -, inhibiteur d'aldose réductase, inhibiteur de 5-lipoxygénase, inhibiteur de dopa-décarboxylase, $\beta$-bloquant adrénergique, anti-trémorique, anti-allergique, analgésique, antibactérien et antifongique, et présentent une large bande d'adsorption dans le rayonnement ultraviolet UVA et UVB, ce qui permet de les utiliser avantageusement comme filtre solaire.

Ainsi, l'oraposide, ses dérivés précités, les extraits de plante en contenant sont particulièrement précieux, notamment dans les domaines pharmaceutique, cosmétique et alimentaire.

Selon un deuxième aspect, la présente invention concerne donc également une composition cosmétique ou pharmaceutique, notamment dermatologique, caractérisée en ce qu'elle comprend, à titre d'ingrédient actif, l'oraposide ou ses dérivés de formule I telle que précédemment définie, dans laquelle un ou plusieurs R représentent indépendamment un atome d'hydrogène, un alkyle inférieur en $C_1$-$C_5$, en particulier méthyle, ou un groupe acyle, en particulier en $C_1$-$C_6$, notamment acétyle, ou un extrait de plante en contenant. De préférence, les dérivés acylés sont tels que seuls les groupes OR portés par les cycles glucopyranosyle ou rhamnopyranosyle sont acylés, notamment acétylés. De préférence encore, les dérivés alkylés sont tels que seuls les groupes OR portés par les cycles phenyle sont alkylés, notamment méthylés.

Selon une variante de réalisation avantageuse, l'oraposide ou ses dérivés précités sont incorporés à une partie comprise entre environ 0,001 et environ 10 % en poids, de préférence entre 0,1 et 5 % en poids de la composition finale.

Suivant un mode de réalisation particulier, la composition selon l'invention est une composition à effet piégeur de radicaux libres, inhibiteur d'aldose réductase, inhibiteur de 5-lipoxygénase, inhibiteur de dopa-décarboxylase, $\beta$-bloquant, anti-trémorique, ou filtrant du rayonnement ultraviolet UVA et UVB.

Selon une variante avantageuse, les compositions de l'invention précédemment définies ne contiennent ni conservateur supplémentaire antibactérien ou antifongique, ni agent antioxydant supplémentaire, notamment contre l'oxydation des lipides de la formule cosmétique ou pharmaceutique.

La formulation sera naturellement adaptée en fonction de l'usage voulu. La composition peut ainsi être formulée sous une forme de dosage injectable, pour une administration orale ou pour un traitement externe par voie topique. L'excipient sera donc adapté à cette formulation et sera un excipient cosmétiquement ou pharmaceutiquement acceptable.

Les compositions selon l'invention sont particulièrement efficaces dans la prévention du vieillissement de la peau et dans le traitement des états inflammatoires qui accompagnent souvent l'érythème solaire. En effet, dans certaines conditions métaboliques ou perturbations d'origine externe comme l'exposition au rayonnement ultraviolet, la réduction de l'oxygène est incomplète et aboutit à la formation de radicaux libres pouvant détériorer les phospholipides des membranes cellulaires. La production de radicaux libres entraîne diverses manifestations physiopathologiques qui participent au vieillissement cellulaire et probablement à la cancérogénèse, au phénomène inflammatoire et au processus d'intoxication hépatique.

Ainsi, les compositions selon l'invention sont destinées en particulier à lutter contre les inflammations, aussi bien en application locale que par voie systémique, notamment les inflammations causées par une exposition excessive au soleil, à lutter contre le vieillissement cellulaire, en particulier contre le vieillissement actinique cutané, et à protéger la peau contre le rayonnement ultraviolet UVA et UVB, à la prévention et au traitement des taches pigmentaires cutanées. Elles sont également efficaces dans le traitement des intoxications hépatiques, quelle que soit leur origine et dans le traitement du diabète, notamment du diabète mellitus, et des troubles du cristallin qui lui sont souvent associés, et comme agent analgésique, antiallergique, psychotrope, anti-parkinsonien et anti-hypertenseur.

Selon un mode de réalisation particulier de l'invention, dans le cadre d'un traitement anti-trémorique, en particulier de la maladie de Parkinson, la composition selon l'invention précitée contient en outre une quantité thérapeutiquement efficace de L-DOPA. En effet, l'oraposide et ses dérivés de formule I précités possèdent une activité de potentialisation de la L-DOPA dans son activité anti-trémorique, efficace en particulier dans la maladie de Parkinson. L'oraposide et ses dérivés de formule I précités associé à la L-DOPA diminuent en effet expérimentalement sur l'animal et sur l'homme les tremblements engendrés par un facteur trémogène. Dans la maladie de Parkinson , on constate une amélioration des mouvements anormaux (de type choréique) et autres troubles secondaires dus au traitement à la L-DOPA.

L'invention sera maintenant décrite en détail en référence à plusieurs exemples de réalisation donnés simplement à titre d'illustration et qui ne sauraient donc limiter la portée de l'invention. Ces exemples sont donnés également en référence à plusieurs figures, objet des dessins annexés, dans lesquels :

- la figure 1 représente la quantité de LDH (lactate déshydrogénase) relarguée par $4.10^6$ hépatocytes (culture de 8 jours) dans le milieu de culture, 3 et 7 h après traitement. La référence 1 des deux blocs d'histogramme concerne les résultats obtenus avec les cultures non traitées, servant de témoins, la référence 2 représente les blocs d'histogramme obtenus avec les cultures traitées au nitroxynil à la concentration $10^{-4}$ M, la référence 3 représente les blocs histogramme obtenus avec les cultures traitées au nitroxynil à la concentration $10^{-4}$ M et au verbascoside à la concentration $5.10^{-5}$ M, la référence 4 représente les blocs histogramme obtenus avec les cultures traitées au nitroxynil à la concentration $10^{-4}$ M et à l'oraposide à la concentration $5.10^{-5}$ M, et la référence 5 représente les blocs histogramme obtenus avec les cultures traitées au nitroxynil à la concentration $10^{-4}$ M et à l'arénarioside à la concentration $5.10^{-5}$ M. Le nitroxynil est un agent hépato-toxique, le verbascoside et l'arénarioside sont d'autres esters hétérosidiques de l'acide caféique connus à l'homme de l'art.
- La figure 2 représente la quantité de LDH (lactate déshydrogénase) relarguée par $4.10^6$ hépatocytes (culture de 24 h) dans le milieu de culture 3, 7 et 24 h après traitement. De même qu'à la figure 1, la référence 1 représente les résultats obtenus avec les cultures non traitées, la référence 2 les résultats obtenus avec les cultures traitées par le chloroforme $10^{-4}$ M, et la référence 3 les résultats obtenus avec les cultures traitées par la combinaison de chloroforme $10^{-4}$ M et d'oraposide à $5.10^{-5}$ M. Le chloroforme est également un agent hépato-toxique.
- La figure 3 représente l'inhibition de la lipoperoxydation sur des microsomes hépatiques par différents composés phénoliques à $5.10^{-5}$ M dans le milieu réactionnel. La référence 1 concerne les blocs histogrammes obtenus avec le lot témoin sans composé phénolique, la référence 2 représente les résultats obtenus avec le verbascoside, la référence 3 représente les résultats obtenusavec l'oraposide, la référence 4 représente les résultats obtenus avec l'arénarioside, la référence 5 représente les résultats obtenus avec l'acide rosmarinique, la référence 6 représente les résultats obtenus avec l'acide isochlorogénique, la référence 7 représente les résultats obtenus avec l'acide chicorique, la référence 8 représente les résultats obtenus avec l'acide caféique et la référence 9 représente les résultats obtenus avec la vitamine E (alpha-tocophérol). Le verbascoside, l'oraposide et l'arénarioside sont des esters hétérosidiques de l'acide caféique et les acides cités sont d'autres esters de l'acide caféique.

- la figure 4 représente l'inhibition de la lipoperoxydation par l'oraposide à différentes concentrations sur des microsomes hépatiques de lapins. La référence 1 représente les résultats obtenus avec le lot témoin sans oraposide, la référence 2 les résultats obtenus avec l'oraposide à la concentration de $5.10^{-5}$ M, la référence 3 les résultats obtenus avec l'oraposide à une concentration de $10^{-5}$ M, la référence 4 les résultats obtenus avec l'oraposide à une concentration de $5.10^{-6}$ M, la référence 5 les résultats obtenus avec l'oraposide à une concentration de $2.10^{-6}$ M, et la référence 6 les résultats obtenus avec l'oraposide à une concentration de $10^{-6}$ M.

Exemple 1

Préparation de l'extrait végétal, purification des cristaux, analyse et établissement de la formule de l'oraposide.

Le matériau de départ est de la poudre de plante (Orobanche) dégraissée à l'éther de pétrole. Cette poudre est mise à macérer dans de l'éthanol à 80 % ou du méthanol à 80 %, à raison de 10 l pour 400 g de poudre de plante, à 50 °C. L'extrait obtenu est concentré et lyophilisé. Cet extrait lyophilisé dit "extrait brut d'orobanche" contient de 3 à 5 % en poids d'oraposide.

Cette macération alcoolique, après filtration, est additionnée rapidement de 20 ml d'une solution aqueuse fraîchement préparée de métabisulfite de sodium à 10 %. A la suite d'un repos d'une nuit à 4 °C, cet extrait est filtré puis concentré de manière à chasser l'alcool. L'extrait devenu aqueux subit un dégraissage définitif par un mélange : éther éthylique débarrassé des peroxydes - éther de pétrole (3 : 1). Cet extrait dégraissé est épuisé par 20 l d'acétate d'éthyle (redistillé sur chlorure de calcium). Les phases "acétate d'éthyle" sont desséchées par du sulfate de sodium (pur, sec), puis réunies et évaporées à sec sous vide. Le résidu poudreux obtenu, de couleur blanc-crème, dit "extrait purifié d'orobanche" contient envirion 30 % en poids d'oraposide. Cet extrait purifié peut être dissous dans de l'eau chaude et la solution mise à cristalliser à la température ambiante puis à + 4 °C pour procéder à une nouvelle purification.

Ces cristaux sont essorés et remis en solution à chaud dans un mélange éthanol 5 % - eau 95 % puis recristallisés à froid. L'opération est répétée plusieurs fois.

Le composant obtenu à l'état de cristaux purs est soumis à diverses analyses classiques, biochimiques et chimico-physiques On détermine le pouvoir rotatoire $(\alpha)^{22D}$ = -110,3 ° (méthanol). Le point de fusion est de 210 °C. Le spectre RMN $^1H$ ($\delta$ en ppm) est :
7,46 et 6,18(2H,2xd, J = 16 Hz, liaison R - CH = CH - R trans), 5(1H,d,J = 1 Hz, H - 1 Rha), 4,92(1H,t,J = 9,5 Hz, H - 4 Glc), 4,57(1H,t,J = 2,5,9 Hz, H - 7' Aglycone), 4,55(1h,d,J = 7,8 Hz, H - 1 Glc), 4,05(1H,t,J = 9,5 Hz, H - 3 Glc), 3,94-3,71(2H,m,J = 12 Hz, 2H - 6 Glc), 3,57(1H,dd,J = 1,3 Hz, H - 2' Rha), 3,45-(1H,m,H - 8' Aglycone), 3,38(1H,dd,J = 7,8,9,5 Hz, H - 2 Glc), 1,05(3H,d,J = 6 Hz, 3H - 1 Rha).

Le spectre UV obtenu avec un appareil de type Kontron Uvikon 860® montre un maximum d'absorption en UVA à 330 nm, et présente en UVB un pic à 290 nm. L'intégration des surfaces des pics UVB permet de comparer l'oraposide au Parsol MCX® où l'on peut observer que l'activité d'absorption UVB de l'oraposide est 0,4 fois celle du Parsol MCX®. Si l'on procède également à une intégration de la surface du pic UVA de l'oraposide que l'on compare au Parsol 1 789®, on observe que l'absorption UVA de l'oraposide est environ 0,2 fois celle du Parsol 1 789®. L'étude des résultats ci-dessus ainsi que des spectres de masse et de rayons X et IR permettent de démontrer que l'oraposide est un 4-caféate de 3-0-($\alpha$-L-rhamnopyranosyl)-[2-(3,4-dihydroxyphényl)-1,2-éthylidène]-$\beta$-D-glucopyranoside.

Exemple 2

Etude in vitro des propriétés de l'oraposide pour la protection des hépatocytes de lapin en culture.

Le modèle expérimental choisi est l'étude de la libération de lactate déshydrogénase (LDH) par les hépatocytes de lapin en culture primaire, cette libération de LDH représentant un marqueur de dégradation des hépatocytes.

Les hépatocytes sont volontairement intoxiqués au nitroxynil ($10^{-4}$ à $10^{-5}$ M) ou au chloroforme (de $10^{-3}$ à $10^{-5}$ M).

L'effet de l'oraposide est évalué pour une concentration de 0,05 mM finale dans le milieu de culture.

La figure 1 montre que l'oraposide diminue très significativement le relargage de LDH par les hépatocytes intoxiqués au nitroxynil et la figure 2 montre un effet moins net sur les cellules traitées au chloroforme mais celui-ci a un effet moins toxique.

L'oraposide par lui-même ne présente aucune toxicité cellulaire, même à une concentration de 100 $\mu$M.

Exemple 3

Etude in vitro de l'inhibition de la lipoperoxydation à partir des microsomes hépatiques de lapin.

Une réaction de lipoperoxydation se produit lorsqu'on incube des microsomes hépatiques de lapin en présence de NADPH.

Les microsomes sont récupérés après traitement des hépatocytes aux ultrasons.

La lipoperoxydation est évaluée par la formation de malonyldialdéhyde selon la méthode de Placer et al., 1966, (Analyt. Biochem. 16, 359-364).

La réaction est effectuée en absence (témoin) et en présence d'acide caféique ou d'oraposide à la concentration de $5.10^{-5}$ M ou en présence d'α-tocophérol (Sigma) à $5.10^{-5}$ M (référence d'activité inhibitrice connue).

La figure 3 montre clairement que l'acide caféique et l'ß-tocophérol ont une activité inhibitrice équivalente et que l'oraposide assure une inhibition plus forte (100 %).

La dose d'oraposide nécessaire pour obtenir une inhibition de 50 % est de $10^{-5}$ M (figure 4).

De plus, il est possible de potentialiser l'oxydation des lipides membranaires par des générateurs de radicaux libres ($CCl_4$ et $H_2O_2$) ; dans ces conditions l'inhibition par l'oraposide est de 100 % avec le $CCl_4$ et de 90 % avec l'$H_2O_2$.

Exemple 4

Mise en évidence d'une activité antibactérienne et antifongique.

On sait qu'un certain nombre de dérivés de l'acide caféique inhibent la multiplication des bactéries et champignons. Cette propriété a été confirmée pour l'oraposide sur 40 souches différentes appartenant à 10 genres de bactéries, ainsi que sur 25 souches différentes appartenant à 25 espèces de champignons.

On observe 100 % d'inhibition de toutes les souches bactériennes dès la dose de 0,5 mg/ml. Les souches bactériennes testées sont : Staphylococcus aureus ; Streptococcus (groupe A et groupe D) ; Escherichia coli ; Klebsiella pneumoniae ; Proteus sp ; Providentia sp ; Seratia marcescens ; Enterobacter cloacae ; Pseudomonas aeruginosa ; Salmonella (S. typhi et S. para-typhi).

Une étude plus détaillée réalisée avec 29 souches bactériennes de Staphyloccus aureus montre une inhibition de 70 % dès la concentration de 0,06 mg/ml et de 80 % à 0,12 mg/ml.

L'activité antifongique a été testée vis-à-vis de contaminents micromicètes couramment rencontrés dans l'alimentation. Le test a été réalisé vis-à-vis de 25 espèces appartenant aux Adelomycètes (ex. Altemaria tenuis ; Fusarim avenceum ; Geotricom candidum...), aux Mucorales (ex. Mucor mucedo, Cunninghamella elegans, Rhizopus nigricans...), aux Endomycetales (ex: Candida lipolytica ; Saccharomyces cerevisiae...), aux Plectomycetes (ex: Aspergillus clavatus, A. niger ; Penicillium chrysogenum, P. rubrum...), aux Pyrenomycetes (ex: Chaetomium globosum, Sordaria himicola...).

| Molécules testées | Souches à croissance inhibée ou diminuée (%) | Souches à croissance inhibée pour une CMI < 10 mg/ml (%) |
|---|---|---|
| Acide rosmarinique | 100 | 96 |
| Oraposide | 92 | 64 |
| Acide caféique | 83 | 52 |
| Acide chlorogénique | 80 | 40 |
| Verbascoside | 72 | 44 |

Exemple 5

Test d'inhibition de l'Aldose-réductase (EC1.1.1.21)( = A.R)

Mode opératoire

On évalue l'inhibition enzymatique in vitro à partir d'un extrait d'enzyme brut isolée de cristallins fraîchement obtenus à partir de bovins et de rats. Le principe de la réaction est le suivant :

$$\text{glycéraldéhyde+NADPH} \underset{}{\overset{A.R.}{\rightleftarrows}} \text{glycérol + NADP}$$

Cette réaction se fait à pH = 6,2 à température ambiante et les mesures d'extinction à 340 nm. Les molécules testées sont dissoutes dans de l'eau bidistillée.

Les résultats obtenus par dosage du glycérol sont exprimés en CI 50 (Concentration permettant d'inhiber 50 % de l'activité enzymatique).

Résultats

Les résultats sont rassemblés dans le tableau suivant :

A.R.-cristallin de veau

| Molécules testées | | C.I.50 (Mole x $L^{-1}$) |
|---|---|---|
| Oraposide | (1) | $1,5 \times 10^{-7}$ |
| | (2) | $1,65 \times 10^{-7}$ |
| Verbascoside | (1) | $1,6 \times 10^{-6}$ |
| | (2) | $1,6 \times 10^{-6}$ |
| Arenarioside | (1) | $2,38 \times 10^{-6}$ |
| | (2) | $1,47 \times 10^{-6}$ |

A.R.-Cristallin de rat

| | |
|---|---|
| Oraposide | $2,20 \times 10^{-7}$ |
| Verbascoside | $1,78 \times 10^{-6}$ |
| Arenarioside | $4,18 \times 10^{-6}$ |

Activité inhibitrice de l'aldose réductase de divers esters hétérosidiques caféiques (1) et (2) correspondent à deux séries d'expériences différentes.

Conclusion

Les quatre molécules sont inhibitrices de l'aldose réductase aussi bien bovine que murine. La molécule la plus active est l'oraposide avec une CI 50 de $1,6 \times 10^{-7}$ mol/l. Elle semble plus active que l'une des molécules les plus prometteuses actuellement sur le marché, à savoir le Sorbinil®, qui est une molécule de synthèse de formule 6-fluorospirochromane-4,4'-imidazoline-2',5'-dione, dont la CI 50 est de $5 \times 10^{-7}$ mol/l.

Exemple 6

Mise en évidence de l'activité anti-radicaux libres de l'oraposide sur des cultures de kératinocytes.

Introduction

L'activité anti-radicaux libres de l'oraposide a été étudiée sur une lignée de kératinocytes humains en culture, en comparaison avec des agents anti-radicaux libres bien connus.

L'activité protectrice de l'agent anti-radicaux libres a été évaluée par la mesure du malonaldéhyde (MDA) dans le milieu de culture (reflet de la lipoperoxydation des lipides de membrane cellulaire) après contact des cellules avec une source de radicaux libres oxygénés (selon la méthode de Placer Z.A. et vol. 1966 Anal. Biochem. 16 359-64, référence précédemment citée).

La cytotoxicité de ces substances est également mesurée par le dosage de la lactate déshydrogénase (LDH) à une concentration élevée (Z. Klin Chem. 1970 8 658 et Klin. Biochem. 1972 10 182).

A la lecture de l'ensemble de ces résultats, un classement des molécules est établi tenant compte à la fois de leur activité anti-radicaux libres et de leur potentiel cytotoxique au niveau cellulaire.

Matériels et méthodes

Matériels :

- Cellules : kératinocytes humains issus d'un carcinome squameux.
- Milieu de culture : milieu de base DMEM (1/2), HAM F12 (1/2) (de la société GIBCO), additionnés de 5 % de sérum de veau foetal (de la société INTERMED).

Méthodes :

- La culture cellulaire et le traitement

Elle est réalisée dans des boîtes de pétri 60 mm (NUNC), densité d'ensemencement : 5 000 cellules par $cm^2$.

Une semaine après l'ensemencement, la monocouche cellulaire à confluence est traitée par 500 l d'une solution contenant un système générateur de radicaux oxygénés composé de Vitamine C $10^{-3}$ M, $FeCl_3$ $10^{-4}$ M, $FeSO_4$-$10^{-4}$ M dans du tampon PBS à pH 8 additionné de la molécule à tester (= milieu réactionnel).

Après une incubation de 2 h à 37 °C, ce milieu d'incubation est récupéré pour le dosage de la MDA.

- Mesure de la LDH

Les composés sont testés à 200 µM dissout dans le DMSO, les cultures témoins contiennent une quantité identique de solvant.

Après 24 h puis pendant 6 j, on détermine la quantité de LDH relarguée dans le milieu de culture après un traitement quotidien par les molécules anti-radicaux libres.

Le surnageant de culture est mis en présence de pyruvate de sodium et de NADH, la cinétique de l'activité LDH est mesurée au spectrophotomètre (Kit BOEHRINGER).

L'activité de la LDH est matérialisée par une augmentation de DO/mn (DO = densité optique) qui reflète donc la cytotoxicité de la molécule.

- Mesure du MDA

Après les 2 h d'incubation avec les cellules, les 500 µl de milieu réactionnel sont prélevés et additonnés d'acide thiobarbiturique pour doser le MDA à 532 nm.

Les résultats sont donnés en % d'inhibition de la production de MDA.

- Les substances testées

- α tocophérol )
- BHT ) substances de référence
- Oraposide

Résultats et discussion

- Cytotoxicité

Elle a été testée à 200 µM pour les 3 produits. Les résultats seront donnés pour les 3 premiers jours de contact et donnés en % d'augmentation de DO par rapport au témoin (+DMSO)

| | J1 | J2 | J3 |
|---|---|---|---|
| α Tocophérol<br>BHT<br>Oraposide | 19 ± 2<br>800 ± 80<br>0 | 83 ± 20<br>111 ± 15<br>0 | 54 ± 8<br>-<br>0 |
| | (moyenne de 3 mesures) | | |

Nous pouvons observer la forte cytotoxicité du BHT qui, dès le premier jour, est responsable d'un fort relarguage de LDH, le phénomène est moins prononcé pour l'α-tocophérol, alors que l'Oraposide ne manifeste aucun effet cytotoxique vis-à-vis des cellules.

- Dosage de la MDA

L'étude a été réalisée à la concentration 100 µM pour les 3 produits après 2 h d'incubation avec le mélange réactionnel. Le MDA produit, suivi par spectrophotométrie donne des résultats exprimés en % d'inhibition de cette production par rapport au témoin (milieu réactionnel seul).

On évalue donc le pouvoir protecteur de la substance anti-radicalaire.

| | | |
|---|---|---|
| α-tocophérol | | 100 % |
| BHT | | 64 ± 2 % |
| Oraposide | (1ère expérience) | 70 ± 3 % |
| | (2ème expérience) | 60 ± 15 %<br>(moyenne des 3 mesures) |

Nous remarquons un pouvoir protecteur de l'Oraposide qui est voisin de celui du BHT, un peu moins puissant que l'α-tocophérol.

Conclusion

Le dosage du MDA dans te surnageant de la culture, nous a permis d'évaluer l'activité anti-radicaux libres de l'oraposide à 100 µM, il se révèle être aussi efficace que le BHT, toutefois, moins que l'α-tocophérol.

Mais il est à noter que contrairement à ces 2 molécules de référence, il ne présente aucun effet cytotoxique jusqu'à 200 µM.

On donne ci-après divers exemples de formulation cosmétique ou pharmaceutique avec l'Oraposide de ces dérivés selon l'invention.

Les pourcentages sont donnés en poids sauf indication contraire.

Exemple 7

Gel après soleil (anti-inflammatoire)

| | |
|---|---|
| - extrait brut d'Orobanche (selon l'exemple 1) | 20 % |
| - gel de Carbopol 940® glycériné à 2,5 % | 70 % |
| - acide hyaluronique | 0,1 % |
| - conservateurs usuels | 0,2 % |
| - parfums | 0,1 % |
| - $H_2O$ q.s.p. | 100 % |

Exemple 8

Crème anti-vieillissement

| | |
|---|---|
| Oraposide encapsulé en liposomes (*) .................... | 25 % |
| Huile de germe de blé .................................. | 10 % |
| Base auto-émulsionnable ................................ | 5 % |
| Eau, contenant conservateurs, parfums, antioxydants usuels, q.s.p. ........................................ | 100 % |

(*) La composition des liposomes contenant l'oraposide est de :

| | |
|---|---|
| - lécithine de soja | 3,6 |
| - β-sitostérol | 0,4 |
| - oraposide | 0,4 |
| - peptide de soja | 1,0 |

- eau quantité suffisante pour 100 %.

L'oraposide étant dans la phase aqueuse.

Exemple 9

Sérum anti-tache

| | |
|---|---|
| - extrait purifié d'Orobanche (selon l'exemple 1) | 5 % |
| - éthanol | 20 % |
| - eau glycérinée | 60 % |
| - substances gélifiantes | 0,6 % |
| - parfums | 0,1 % |
| - conservateurs | 0,2 % |
| - eau q.s.p. | 100 % |

Exemple 10

Protecteurs solaires

| | |
|---|---|
| - extrait purifié d'Orobanche (selon l'exemple 1) | 10 % |
| - titane micronisé | 2 % |
| - émulsionnants non ioniques | 5 % |
| - huiles | 30 % |
| - gel neutralisé Carbopol 940® à 2,5 % | 0,2 % |
| - silicone | 5 % |
| - eau contenant conservateurs, antioxydants, parfums q.s.p. | 100 %. |

Exemple 11

Composition pharmaceutique

On prépare des gélules, destinées à une administration orale, dosées à 60 mg d'oraposide ou de ses dérivés de formule I précités, dans un excipient usuel pour gélule.

On pourra utiliser ces gélules, notamment pour le traitement de la maladie de Parkinson à une posologie de 4 à 6 gélules par jour, ce qui correspond en général à 1 à 6 mg d'oraposide pur par kg du poids du corps par jour.

L'invention couvre encore un procédé de préparation d'une composition cosmétique ou pharmaceutique, notamment dermatologique, caractérisé en ce qu'il comprend l'incorporation d'une quantité efficace de l'oraposide ou de ses dérivés précites dans un excipient, support ou véhicule cosmétiquement ou pharmaceutiquement acceptable. De préférence, on incorpore entre 0,001 % et environ 10 % en poids, encore mieux entre environ 0,1 et 5 % en poids, d'oraposide ou ses dérivés dans ladite composition, en particulier dans le cadre d'une application topique.

L'invention couvre encore un procédé de traitement d'affections provoquées par des radicaux libres quelle que soit leur origine, caractérisé en ce qu'il comprend l'administration au sujet souffrant de l'une desdites affections, sous une forme appropriée, d'une quantité efficace d'oraposide ou de ses dérivés de formule I précitée.

Selon une variante de réalisation de ce procédé de traitament, en particulier dans le cadre d'une application topique, on administre une composition dosée à 0,001 à 10 % en poids, ce préférence de 0,01 à 5 % en poids, en oraposide ou en ses dérivés précités.

Selon une variante de réalisation particulière, on traite les inflammations, notamment les inflammations causées par une exposition excessive au soleil, aussi bien en application locale que par voie orale, rectale, ou par injection, d'une quantité efficace pour obtenir l'effet désiré d'oraposide ou de l'un de ses dérivés de formule I précités.

Selon une autre variante, on prévient ou on traite le vieillissement cellulaire, en particulier le vieillissement cutané et notamment actinique par application topique d'une quantité efficace pour obtenir l'effet désiré d'oraposide ou de l'un de ses dérivés de formule I précités.

Selon encore une autre variante, on réalise la protection de la peau contre le rayonnement ultraviolet UVA et UVB, par aplication topique d'une quantité efficace pour obtenir l'effet de protection ultraviolet désiré, d'oraposide ou de l'un de ses dérivés de formule I précités.

Selon encore une autre variante, on prévient ou on traite les taches pigmentaires cutanées par application topique d'une quantité efficace d'oraposide ou de l'un de ses dérivés de formule I précités.

Selon encore une autre variante de réalisation, on réalise le traitement des intoxications hépatiques quelle que soit leur origine, par application de préférence par voie orale ou par injection, d'une quantité efficace pour obtenir l'effet désiré d'oraposide ou de l'un de ses dérivés de formule I précités.

Selon encore une autre variante de réalisation, on réalise le traitement du diabète, notamment du diabète mellitus, ou des troubles du cristallin qui lui sont associés, en administrant au sujet souffrant de ladite affection, sous une forme appropriée, une quantité efficace d'oraposide ou de l'un de ses dérivés de formule I précités.

Selon encore une autre variante de réalisation, on réalise le traitement de la douleur en administrant au sujet souffrant de ladite douleur, sous une forme appropriée, une quantité efficace d'oraposide ou de l'un de ses dérivés de formule I précités.

Selon encore une autre variante de réalisation, on réalise un traitement anti-allergique en administrant au sujet souffrant d'une allergie, sous une forme appropriée, une quantité efficace d'oraposide ou de l'un de ses dérivés de formule I précités.

Selon encore une autre variante de réalisation, on réalise un traitement de la maladie de Parkinson, en administrant au sujet souffrant de ladite maladie, sous une forme apppropriée, une quantité efficace d'oraposide et de ses dérivés de formule I précités, de préférence en combinaison avec la L-DOPA.

Selon encore une autre variante de réalisation, on réalise un traitement anti-hypertenseur, en administrant au sujet souffrant d'hypertension artérielle, et sous une forme appropriée, une quantité efficace d'oraposide ou de l'un de ses dérivés de formule I précités.

Selon encore une autre variante de réalisation, on réalise un traitement anti-trémorique, en administrant au sujet souffrant de tremblements, en particulier de tremblements dus à la maladie de Parkinson, et sous une forme appropriée, une quantité efficace d'oraposide ou de l'un de ses dérivés de formule I précités.

Selon encore une autre variante de réalisation, on administre à un sujet, sous une forme appropriée, une quantité efficace pour l'obtention d'un effet psychotrope, d'oraposide ou de l'un de ses dérivés de

formule I précités.

## Revendications

**Revendications pour les Etats contractants suivants : AT, BE, CH, LI, DE, DK, ES, GB, GR, IT, LU, NL, SE**

1. Nouveau dérivé de l'acide caféique, caractérisé en ce qu'il s'agit de l'oraposide ou de ses dérivés de formule I suivante :

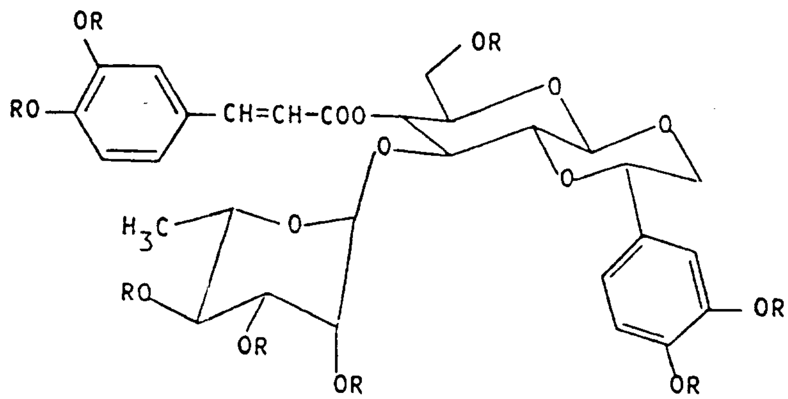

(I)

dans laquelle un ou plusieurs R représentent indépendamment un atome d'hydrogène, un groupe alkyle inférieur en $C_1$-$C_5$, en particulier méthyle, ou un groupe acyle, en particulier en $C_1$-$C_6$, notamment acétyle.

2. Composé selon la revendication 1, caractérisé en ce que seuls les groupes OR portés par les cycles glucopyranosyle ou rhamnopyranosyle sont acylés, notamment acétylés.

3. Composé selon la revendication 1 ou 2, caractérisé en ce que seuls les groupes OR portés par les cycles phényle sont alkylés, notamment méthylés.

4. Composé selon la revendication 1 à 3, caractérisé en ce que l'oraposide ou ses dérivés sont extraits à partir d'une plante de la famille des Orobanchacées, en particulier Orobanche rapum genistae.

5. Composition cosmétique ou pharmaceutique, notamment dermatologique, caractérisée en ce qu'elle comprend à titre d'ingrédient actif l'oraposide ou ses dérivés de formule I tels que définis à l'une des revendications 1 à 4.

6. Composition selon la revendication 4, caractérisée en ce qu'elle comprend d'environ 0,001 à environ 10 % en poids, de préférence environ 0,1 à environ 5 % en poids, de l'oraposide ou de ses dérivés précités, par rapport au poids total de la composition finale.

7. Utilisation de l'oraposide ou de ses dérivés de formule I tels que définis à l'une des revendications 1 à 4, pour la fabrication d'une composition cosmétique ou pharmaceutique, notamment dermatologique, ayant une activité anti-radicaux libres.

8. Utilisation de l'oraposide ou de ses dérivés de formule I tels que définis à l'une des revendications 1 à 4, pour la fabrication d'une composition cosmétique ou pharmaceutique, notamment dermatologique, ayant une activité anti-inflammatoire.

9. Utilisation de l'oraposide ou de ses dérivés de formule I tels que définis à l'une des revendications 1 à 4, pour la fabrication d'une composition pharmaceutique ayant une activité hépatoprotectrice.

**10.** Utilisation de l'oraposide ou de ses dérivés de formule I tels que définis à l'une des revendications 1 à 4, pour la fabrication d'une composition cosmétique ou pharmaceutique, notamment dermatologique, ayant une activité antivieillissement cellulaire, en particulier antivieillissement des cellules de la peau et notamment antivieillissement actinique.

**11.** Utilisation de l'oraposide ou de ses dérivés de formule I tels que définis à l'une des revendications 1 à 4, pour la fabrication d'une composition cosmétique ou pharmaceutique, notamment dermatologique, ayant une activité de prévention ou de traitement des taches pigmentaires cutanées.

**12.** Utilisation de l'oraposide ou de ses dérivés de formule I tels que définis à l'une des revendications 1 à 4, pour la fabrication d'une composition pharmaceutique ayant une activité inhibitrice d'aldose réductase.

**13.** Utilisation de l'oraposide ou de ses dérivés de formule I tels que définis à l'une des revendications 1 à 4, pour la fabrication d'une composition cosmétique ou pharmaceutique, notamment dermatologique, ayant une activité inhibitrice de 5-lipoxygénase.

**14.** Utilisation de l'oraposide ou de ses dérivés de formule I tels que définis à l'une des revendications 1 à 4, pour la fabrication d'une composition cosmétique ou pharmaceutique, notamment dermatologique, ayant une activité inhibitrice de dopa-décarboxylase.

**15.** Utilisation de l'oraposide ou de ses dérivés de formule I tels que définis à l'une des revendications 1 à 4, pour la fabrication d'une composition pharmaceutique ayant une activité $\beta$-bloquante.

**16.** Utilisation de l'oraposide ou de ses dérivés de formule I tels que définis à l'une des revendications 1 à 4, pour la fabrication d'une composition cosmétique ou pharmaceutique, notamment dermatologique, ayant une activité de filtration du rayonnement ultraviolet UVA et UVB.

**17.** Utilisation de l'oraposide ou de ses dérivés de formule I tels que définis à l'une des revendications 1 à 4, pour la fabrication d'une composition cosmétique ou pharmaceutique, notamment dermatologique, ayant une activité analgésique.

**18.** Utilisation de l'oraposide ou de ses dérivés de formule I tels que définis à l'une des revendications 1 à 4, pour la fabrication d'une composition cosmétique ou pharmaceutique, notamment dermatologique, ayant une activité anti-allergique.

**19.** Utilisation de l'oraposide ou de ses dérivés de formule I tels que définis à l'une des revendications 1 à 4, pour la fabrication d'une composition pharmaceutique ayant une activité anti-trémorique.

**20.** Utilisation de l'oraposide ou de ses dérivés de formule I tels que définis à l'une des revendications 1 à 4, pour la fabrication d'une composition pharmaceutique ayant une activité psychotrope.

**21.** Utilisation de l'oraposide ou de ses dérivés de formule I tels que définis à l'une des revendications 1 à 4, pour la fabrication d'une composition pharmaceutique ayant un effet anti-hypertenseur.

**22.** Utilisation selon l'une des revendications 7 à 21, caractérisée en ce que la composition n'est pas additionnée d'agent conservateur antibactérien ou antifongique et/ou d'agent conservateur antioxydant, en raison de l'activité antibactérienne ou antioxydante de l'oraposide ou ses dérivés.

**Revendications pour l'Etat contractant suivant : FR**

**1.** Nouveau dérivé de l'acide caféique, caractérisé en ce qu'il s'agit des dérivés de l'oraposide de formule I suivante :

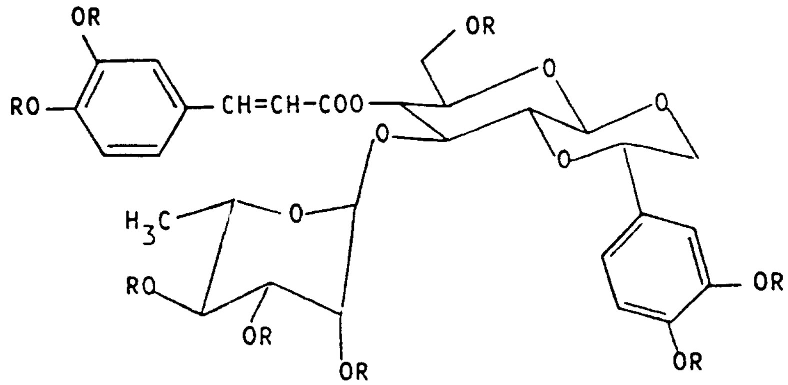

(I)

dans laquelle un ou plusieurs R représentent indépendamment un atome d'hydrogène, un groupe alkyle inférieur en $C_1$-$C_5$, en particulier méthyle, ou un groupe acyle, en particulier en $C_1$-$C_6$, notamment acétyle, à l'exclusion du cas où tous les R représentent simultanément un atome d'hydrogène.

2. Composé selon la revendication 1, caractérisé en ce que seuls les groupes OR portés par les cycles glucopyranosyle ou rhamnopyranosyle sont acylés, notamment acétylés.

3. Composé selon la revendication 1 ou 2, caractérisé en ce que seuls les groupes OR portés par les cycles phényle sont alkylés, notamment méthylés.

4. Composé selon la revendication 1 à 3, caractérisé en ce que les dérivés de l'oraposide sont extraits à partir d'une plante de la famille des Orobanchacées, en particulier Orobanche rapum genistae.

5. Composition cosmétique ou pharmaceutique, notamment dermatologique, caractérisée en ce qu'elle comprend à titre d'ingrédient actif les dérivés de l'oraposide de formule I tels que définis à l'une des revendications 1 à 4.

6. Composition selon la revendication 4, caractérisée en ce qu'elle comprend d'environ 0,001 à environ 10 % en poids, de préférence environ 0,1 à environ 5 % en poids, de dérivés de l'oraposide précités, par rapport au poids total de la composition finale.

7. Composition selon la revendication 5 ou 6, caractérisée en ce qu'elle n'est pas additionnée d'agent conservateur antibactérien ou antifongique et/ou d'agent conservateur anti-oxydant, en raison de l'activité antibactérienne ou antioxydante de l'oraposide ou ses dérivés.

8. Utilisation des dérivés de l'oraposide de formule I tels que définis à l'une des revendications 1 à 4 pour la fabrication d'une composition cosmétique ou pharmaceutique, notamment dermatologique, ayant une activité anti-radicaux libres, une activité anti-inflammatoire, une activité hépatoprotectrice, une activité antivieillissement cellulaire, en particulier antivieillissement des cellules de la peau et notamment antivieillissement actinique.

**9.** Utilisation de l'oraposide ou de ses dérivés de formule I suivante :

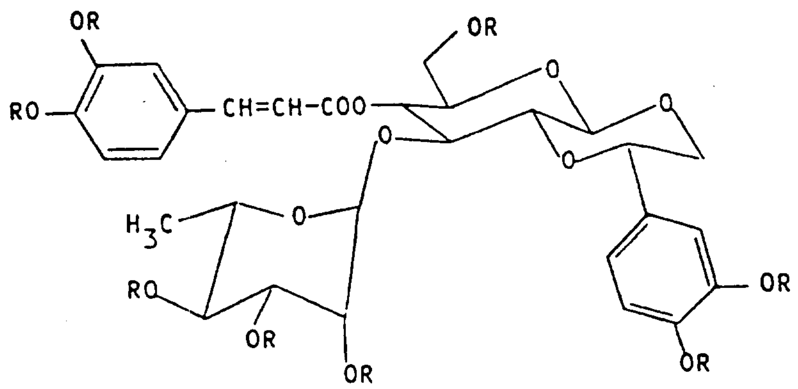

(I)

dans laquelle un ou plusieurs R représentent indépendamment un atome d'hydrogène, un groupe alkyle inférieur en $C_1$-$C_5$, en particulier méthyle, ou un groupe acyle, en particulier en $C_1$-$C_6$, notamment acétyle, pour la fabrication d'une composition cosmétique ou pharmaceutique, notamment dermatologique, ayant une activité de prévention ou de traitement des taches pigmentaires cutanées, une activité inhibitrice d'aldose réductase, une activité inhibitrice de 5-lipoxygénase, une activité inhibitrice de dopa-décarboxylase, une activité $\beta$-bloquante, une activité de filtration du rayonnement ultraviolet UVA et UVB, une activité analgésique, une activité anti-allergique, une activité anti-trémorique, une activité psychotrope, un effet anti-hypertenseur.

**10.** Utilisation de l'oraposide ou de ses dérivés de formule I tels que définis à la revendication 9, pour la fabrication d'une composition cosmétique ou pharmaceutique, notamment dermatologique, ayant une activité anti-radicaux libres.

**11.** Utilisation de l'oraposide ou de ses dérivés de formule I tels que définis à la revendication 9, pour la fabrication d'une composition cosmétique ou pharmaceutique, notamment dermatologique, ayant une activité anti-inflammatoire.

**12.** Utilisation de l'oraposide ou de ses dérivés de formule I tels que définis à la revendication 9, pour la fabrication d'une composition pharmaceutique ayant une activité hépatoprotectrice.

**13.** Utilisation de l'oraposide ou de ses dérivés de formule I tels que définis à la revendication 9, pour la fabrication d'une composition cosmétique ou pharmaceutique, notamment dermatologique, ayant une activité antivieillissement cellulaire, en particulier antivieillissement des cellules de la peau et notamment antivieillissement actinique.

**14.** Utilisation de l'oraposide ou de ses dérivés de formule I tels que définis à la revendication 9, pour la fabrication d'une composition cosmétique ou pharmaceutique, notamment dermatologique, ayant une activité de prévention ou de traitement des taches pigmentaires cutanées.

**15.** Utilisation de l'oraposide ou de ses dérivés de formule I tels que définis à la revendication 9, pour la fabrication d'une composition pharmaceutique ayant une activité inhibitrice d'aldose réductase.

**16.** Utilisation de l'oraposide ou de ses dérivés de formule I tels que définis à la revendication 9, pour la fabrication d'une composition cosmétique ou pharmaceutique, notamment dermatologique, ayant une activité inhibitrice de 5-lipoxygénase.

**17.** Utilisation de l'oraposide ou de ses dérivés de formule I tels que définis à la revendication 9, pour la fabrication d'une composition cosmétique ou pharmaceutique, notamment dermatologique, ayant une activité inhibitrice de dopa-décarboxylase.

**18.** Utilisation de l'oraposide ou de ses dérivés de formule I tels que définis à la revendication 9, pour la fabrication d'une composition pharmaceutique ayant une activité $\beta$-bloquante.

**19.** Utilisation de l'oraposide ou de ses dérivés de formule I tels que définis à la revendication 9, pour la fabrication d'une composition cosmétique ou pharmaceutique, notamment dermatologique, ayant une activité de filtration du rayonnement ultraviolet UVA et UVB.

**20.** Utilisation de l'oraposide ou de ses dérivés de formule I tels que définis à la revendication 9, pour la fabrication d'une composition cosmétique ou pharmaceutique, notamment dermatologique, ayant une activité analgésique.

**21.** Utilisation de l'oraposide ou de ses dérivés de formule I tels que définis à la revendication 9, pour la fabrication d'une composition cosmétique ou pharmaceutique, notamment dermatologique, ayant une activité anti-allergique.

**22.** Utilisation de l'oraposide ou de ses dérivés de formule I tels que définis à la revendication 9, pour la fabrication d'une composition pharmaceutique ayant une activité anti-trémorique.

**23.** Utilisation de l'oraposide ou de ses dérivés de formule I tels que définis à la revendication 9, pour la fabrication d'une composition pharmaceutique ayant une activité psychotrope.

**24.** Utilisation de l'oraposide ou de ses dérivés de formule I tels que définis à la revendication 9 pour la fabrication d'une composition pharmaceutique ayant un effet anti-hypertenseur.

**Claims**

**Claims for the following Contracting States : AT, BE, CH, LI, DE, DK, ES, GB, GR, IT, LU, NL, SE**

**1.** New derivative of caffeic acid, characterized in that it is the oraposide or its derivatives of formula I below:

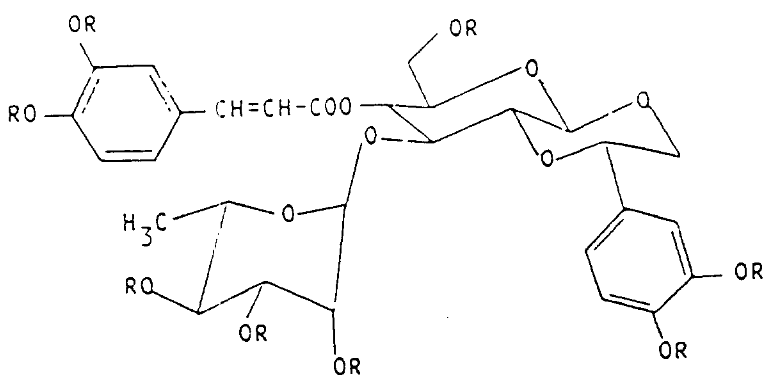

(I)

in which one or more R independently represent a hydrogen atom, a lower $C_1$-$C_5$ alkyl group, in particular methyl, or an acyl group, in particular a $C_1$-$C_6$ group, in particular acetyl.

**2.** Compound according to claim 1, characterized in that only the OR groups on the glucopyranosyl or rhamnopyranosyl rings are acylated, in particular acetylated.

**3.** Compound according to claim 1 or 2, characterized in that only the OR groups on the phenyl rings are alkylated, in particular methylated.

**4.** Compound according to claims 1 to 3, characterized in that the oraposide or its derivatives are extracted from a plant of the family of the Orobanchacae, in particular the broomrape, *Rapum genistae*.

5. Cosmetic or pharmaceutical composition, in particular a dermatological composition, characterized in that it comprises oraposide or its derivatives of formula I as defined in one of claims 1 to 4 as the active ingredient.

6. Composition according to claim 4, characterized in that it comprises approximately 0.001 to approximately 10% by weight, preferably from approximately 0.1 to approximately 5% by weight, of oraposide or its aforesaid derivatives, with respect to the total weight of the final composition.

7. Use of the oraposide or of its derivatives of formula I as defined in one of claims 1 to 4, for the manufacture of a cosmetic or pharmaceutical composition, in particular a dermatological composition, having anti-free-radical activity.

8. Use of the oraposide or of its derivatives of formula I as defined in one of claims 1 to 4, for the manufacture of a cosmetic or pharmaceutical composition, in particular a dermatological composition, having anti-inflammatory activity.

9. Use of the oraposide or of its derivatives of formula I as defined in one of claims 1 to 4, for the manufacture of a pharmaceutical composition having hepatoprotective activity.

10. Use of the oraposide or of its derivatives of formula I as defined in one of claims 1 to 4, for the manufacture of a cosmetic or pharmaceutical composition, in particular a dermatological composition, having anti-cell-aging activity, in particular against the aging of skin cells and in particular against aging due to actinic radiation exposure.

11. Use of the oraposide or of its derivatives of formula I as defined in one of claims 1 to 4, for the manufacture of a cosmetic or pharmaceutical composition, in particular a dermatological composition, having the effect of preventing or treating pigmented skin patches.

12. Use of the oraposide or of its derivatives of formula I as defined in one of claims 1 to 4, for the manufacture of a pharmaceutical composition having aldose reductase inhibiting activity.

13. Use of the oraposide or of its derivatives of formula I as defined in one of claims 1 to 4, for the manufacture of a cosmetic or pharmaceutical composition, in particular a dermatological composition, having 5-lipoxygenase inhibiting activity.

14. Use of the oraposide or of its derivatives of formula I as defined in one of claims 1 to 4, for the manufacture of a cosmetic or pharmaceutical composition, in particular a dermatological composition, having dopa-decarboxylase inhibiting activity.

15. Use of the oraposide or of its derivatives of formula I as defined in one of claims 1 to 4, for the manufacture of a pharmaceutical composition having $\beta$-blocking activity.

16. Use of the oraposide or of its derivatives of formula I as defined in one of claims 1 to 4, for the manufacture of a cosmetic or pharmaceutical composition, in particular a dermatological composition, having the property of filtering ultraviolet UVA and UVB radiation.

17. Use of the oraposide or of its derivatives of formula I as defined in one of claims 1 to 4, for the manufacture of a cosmetic or pharmaceutical composition, in particular a dermatological composition, having analgesic activity.

18. Use of the oraposide or of its derivatives of formula I as defined in one of claims 1 to 4, for the manufacture of a cosmetic or pharmaceutical composition, in particular a dermatological composition, having anti-allergic activity.

19. Use of the oraposide or of its derivatives of formula I as defined in one of claims 1 to 4, for the manufacture of a pharmaceutical composition having anti-tremor activity.

20. Use of the oraposide or of its derivatives of formula I as defined in one of claims 1 to 4, for the manufacture of a pharmaceutical composition having psychotropic activity.

21. Use of the oraposide or of its derivatives of formula I as defined in one of claims 1 to 4, for the manufacture of a pharmaceutical composition having an antihypertensive activity.

22. Use according to one of claims 7 to 21, characterized in that no antibacterial or antifungal preservative and/or antioxidant preservative is added to the composition because of the antibacterial or antioxidant activity of the oraposide or its derivatives.

**Claims for the following Contracting State : FR**

1. New derivative of caffeic acid, characterized in that it is the oraposide derivatives of formula I below:

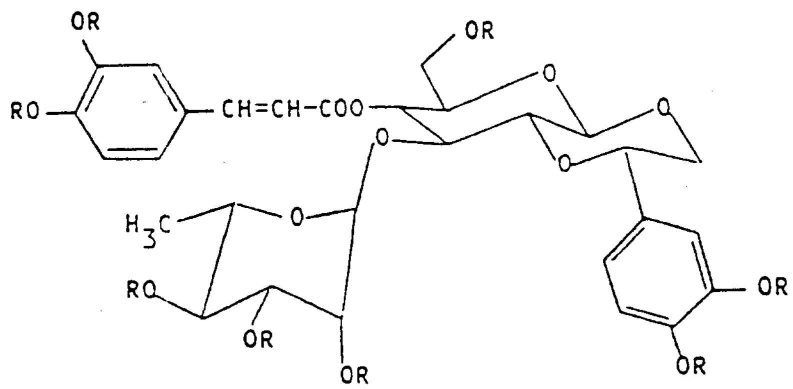

(I)

in which one or more R independently represent a hydrogen atom, a lower $C_1$-$C_5$ alkyl group, in particular methyl, or an acyl group, in particular a $C_1$-$C_6$ group, in particular acetyl, with the exception of the case where all the Rs simultaneously represent a hydrogen atom.

2. Compound according to claim 1, characterized in that only the OR groups on the glucopyranosyl or rhamnopyranosyl rings are acylated, in particular acetylated.

3. Compound according to claim 1 or 2, characterized in that only the OR groups on the phenyl rings are alkylated, in particular methylated.

4. Compound according to claims 1 to 3, characterized in that the oraposide derivatives are extracted from a plant of the family of the Orobanchacae, in particular the broomrape, *Rapum genistae*.

5. Cosmetic or pharmaceutical composition, in particular a dermatological composition, characterized in that it comprises the oraposide derivatives of formula I as defined in one of claims 1 to 4 as the active ingredient.

6. Composition according to claim 4, characterized in that it comprises approximately 0.001 to approximately 10% by weight, preferably from approximately 0.1 to approximately 5% by weight, of the aforesaid oraposide derivatives, with respect to the total weight of the final composition.

7. Composition according to claim 5 or 6, characterized in that no antibacterial or antifungal preservative and/or antioxidant preservative is added thereto because of the antibacterial or antioxidant activity of the oraposide or its derivatives.

8. Use of the oraposide derivatives of formula I as defined in one of claims 1 to 4, for the manufacture of a cosmetic or pharmaceutical composition, in particular a dermatological composition, having anti-free-radical activity, anti-inflammatory activity, hepatoprotective activity, anti-cell-aging activity, in particular against the aging of skin cells and in particular against aging due to actinic radiation exposure.

**9.** Use of the oraposide or of its derivatives of formula I below:

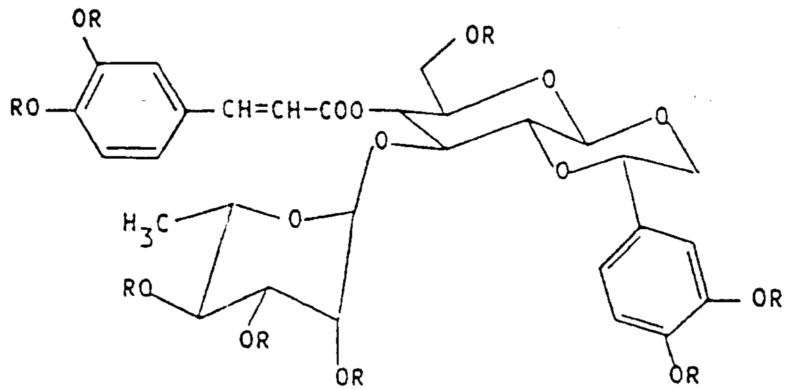

(I)

in which one or more Rs independently represent a hydrogen atom, a lower $C_1$-$C_5$ alkyl group, in particular methyl, or an acyl group, in particular a $C_1$-$C_6$ group, in particular acetyl, for the manufacture of a cosmetic or pharmaceutical composition, in particular a dermatological composition, having the effect of preventing or treating pigmented skin patches, aldose reductase inhibiting activity, 5-lipoxygenase inhibiting activity, dopa-decarboxylase inhibiting activity, β-blocking activity, filtering ultraviolet UVA and UVB radiation, analgesic activity, anti-allergic activity, anti-tremor activity, psychotropic activity, antihypertensive activity.

**10.** Use of the oraposide or of its derivatives of formula I as defined in claim 9, for the manufacture of a cosmetic or pharmaceutical composition, in particular a dermatological composition, having anti-free-radical activity.

**11.** Use of the oraposide or of its derivatives of formula I as defined in claim 9, for the manufacture of a cosmetic or pharmaceutical composition, in particular a dermatological composition, having anti-inflammatory activity.

**12.** Use of the oraposide or of its derivatives of formula I as defined in claim 9, for the manufacture of a pharmaceutical composition having hepatoprotective activity.

**13.** Use of the oraposide or of its derivatives of formula I as defined in claim 9, for the manufacture of a cosmetic or pharmaceutical composition, in particular a dermatological composition, having anti-cell-aging activity, in particular against the aging of skin cells and in particular against aging due to actinic radiation exposure.

**14.** Use of the oraposide or of its derivatives of formula I as defined in claim 9, for the manufacture of a cosmetic or pharmaceutical composition, in particular a dermatological composition, having the effect of preventing or treating pigmented skin patches.

**15.** Use of the oraposide or of its derivatives of formula I as defined in claim 9, for the manufacture of a pharmaceutical composition having aldose reductase inhibiting activity.

**16.** Use of the oraposide or of its derivatives of formula I as defined in claim 9, for the manufacture of a cosmetic or pharmaceutical composition, in particular a dermatological composition, having 5-lipoxygenase inhibiting activity.

**17.** Use of the oraposide or of its derivatives of formula I as defined in claim 9, for the manufacture of a cosmetic or pharmaceutical composition, in particular a dermatological composition, having dopa-decarboxylase inhibiting activity.

**18.** Use of the oraposide or of its derivatives of formula I as defined in claim 9, for the manufacture of a pharmaceutical composition having β-blocking activity.

**19.** Use of the oraposide or of its derivatives of formula I as defined in claim 9, for the manufacture of a cosmetic or pharmaceutical composition, in particular a dermatological composition, having the property of filtering ultraviolet UVA and UVB radiation.

**20.** Use of the oraposide or of its derivatives of formula I as defined in claim 9, for the manufacture of a cosmetic or pharmaceutical composition, in particular a dermatological composition, having analgesic activity.

**21.** Use of the oraposide or of its derivatives of formula I as defined in claim 9, for the manufacture of a cosmetic or pharmaceutical composition, in particular a dermatological composition, having anti-allergic activity.

**22.** Use of the oraposide or of its derivatives of formula I as defined in claim 9, for the manufacture of a pharmaceutical composition having anti-tremor activity.

**23.** Use of the oraposide or of its derivatives of formula I as defined in claim 9, for the manufacture of a pharmaceutical composition having psychotropic activity.

**24.** Use of the oraposide or of its derivatives of formula I as defined in claim 9, for the manufacture of a pharmaceutical composition having an antihypertensive activity.

**Patentansprüche**
**Patentansprüche für folgende Vertragsstaaten : AT, BE, CH, LI, DE, DK, ES, GB, GR, IT, LU, NL, SE**

**1.** Neues Kaffeesäure-Derivat, dadurch gekennzeichnet, daß es sich handelt um Oraposid oder seine Derivate der folgenden Formel (I):

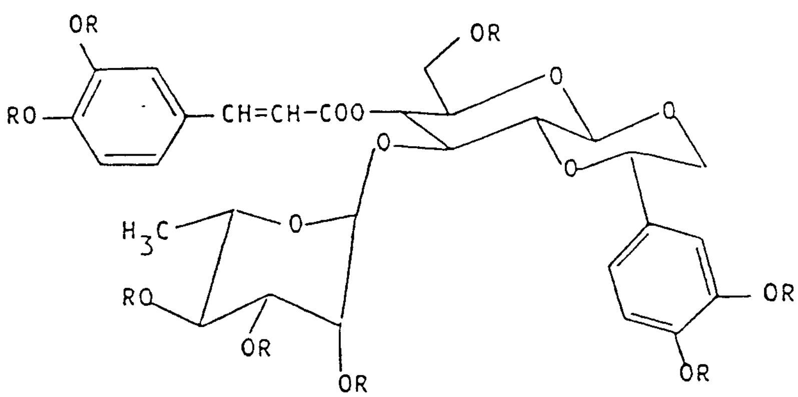

(I),

worin eines oder mehrere R unabhängig ein Wasserstoffatom, eine Alkyl-Gruppe, niederer als $C_1$-$C_5$, insbesondere Methyl, oder eine Acyl-Gruppe, insbesondere $C_1$-$C_6$, spezifisch Acetyl, bedeuten.

**2.** Verbindung nach Anspruch 1, dadurch gekennzeichnet, daß nur die OR-Gruppen, die von den Glucopyranosyl- oder Rhamnopyranosyl-Ringen getragen werden, acyliert, insbesondere acetyliert sind.

**3.** Verbindung nach Anspruch 1 oder 2, dadurch gekennzeichnet, daß nur die OR-Gruppen, die von den Phenyl-Ringen getragen werden, alkyliert, insbesondere methyliert sind.

**4.** Verbindung nach Anspruch 1 bis 3, dadurch gekennzeichnet, daß das Oraposid oder seine Derivate aus einer Pflanze der Familie Orobanchaceae, insbesondere Orobanche rapum genistae, extrahiert werden.

**5.** Kosmetische oder pharmazeutische, insbesondere dermatologische Zusammensetzung, dadurch gekennzeichnet, daß diese als aktiven Bestandteil Oraposid oder seine Derivate der Formel (I), wie in einem der Ansprüche 1 bis 4 definiert, umfaßt.

**6.** Zusammensetzung nach Anspruch 4, dadurch gekennzeichnet, daß diese etwa 0,001 bis etwa 10 Masse-%, vorzugsweise etwa 0,1 bis etwa 5 Masse-%, Oraposid oder seiner Derivate, bezogen auf die Gesamtmasse der End-Zusammensetzung, umfaßt.

**7.** Verwendung von Oraposid oder seinen Derivaten der Formel (I), wie in einem der Ansprüche 1 bis 4 definiert, bei der Herstellung einer kosmetischen oder pharmazeutischen, insbesondere dermatologischen Zusammensetzung mit Wirksamkeit gegen freie Radikale.

**8.** Verwendung von Oraposid oder seinen Derivaten der Formel (I), wie in einem der Ansprüche 1 bis 4 definiert, bei der Herstellung einer kosmetischen oder pharmazeutischen, insbesondere dermatologischen Zusammensetzung mit entzündungshemmender Wirksamkeit.

**9.** Verwendung von Oraposid oder seinen Derivaten der Formel (I), wie in einem der Ansprüche 1 bis 4 definiert, bei der Herstellung einer pharmazeutischen Zusammensetzung mit Hepato-Schutzwirksamkeit.

**10.** Verwendung von Oraposid oder seinen Derivaten der Formel (I), wie in einem der Ansprüche 1 bis 4 definiert, bei der Herstellung einer kosmetischen oder pharmazeutischen, insbesondere dermatologischen Zusammensetzung mit Wirksamkeit gegen die Alterung von Zellen, insbesondere gegen die Alterung von Hautzellen und spezifisch gegen die Alterung durch aktinische Strahlung.

**11.** Verwendung von Oraposid oder seinen Derivaten der Formel (I), wie in einem der Ansprüche 1 bis 4 definiert, bei der Herstellung einer kosmetischen oder pharmazeutischen, insbesondere dermatologischen Zusammensetzung mit Wirksamkeit bei der Prävention oder Behandlung von Hautpigmentflekken.

**12.** Verwendung von Oraposid oder seinen Derivaten der Formel (I), wie in einem der Ansprüche 1 bis 4 definiert, bei der Herstellung einer pharmazeutischen Zusammensetzung mit Aldosereduktase-Inhibitor-Wirksamkeit.

**13.** Verwendung von Oraposid oder seinen Derivaten der Formel (I), wie in einem der Ansprüche 1 bis 4 definiert, bei der Herstellung einer kosmetischen oder pharmazeutischen, insbesondere dermatologischen Zusammensetzung mit 5-Lipoxygenase-Wirksamkeit.

**14.** Verwendung von Oraposid oder seinen Derivaten der Formel (I), wie in einem der Ansprüche 1 bis 4 definiert, bei der Herstellung einer kosmetischen oder pharmazeutischen, insbesondere dermatologischen Zusammensetzung mit DOPA-Decarboxylase-Inhibitor-Wirksamkeit.

**15.** Verwendung von Oraposid oder seinen Derivaten der Formel (I), wie in einem der Ansprüche 1 bis 4 definiert, bei der Herstellung einer pharmazeutischen Zusammensetzung mit $\beta$-Blocker-Wirksamkeit.

**16.** Verwendung von Oraposid oder seinen Derivaten der Formel (I), wie in einem der Ansprüche 1 bis 4 definiert, bei der Herstellung einer kosmetischen oder pharmazeutischen, insbesondere dermatologischen Zusammensetzung mit Wirksamkeit bei der Filterung von UVA- oder UVB-Ultraviolettstrahlung.

**17.** Verwendung von Oraposid oder seinen Derivaten der Formel (I), wie in einem der Ansprüche 1 bis 4 definiert, bei der Herstellung einer kosmetischen oder pharmazeutischen, insbesondere dermatologischen Zusammensetzung mit analgetischer Wirksamkeit.

**18.** Verwendung von Oraposid oder seinen Derivaten der Formel (I), wie in einem der Ansprüche 1 bis 4 definiert, bei der Herstellung einer kosmetischen oder pharmazeutischen, insbesondere dermatologischen Zusammensetzung mit anti-allergischer Wirksamkeit.

**19.** Verwendung von Oraposid oder seinen Derivaten der Formel (I), wie in einem der Ansprüche 1 bis 4 definiert, bei der Herstellung einer pharmazeutischen Zusammensetzung mit Anti-Tremor-Wirksamkeit.

**20.** Verwendung von Oraposid oder seinen Derivaten der Formel (I), wie in einem der Ansprüche 1 bis 4 definiert, bei der Herstellung einer pharmazeutischen Zusammensetzung mit psychotroper Wirksamkeit.

**21.** Verwendung von Oraposid oder seinen Derivaten der Formel (I), wie in einem der Ansprüche 1 bis 4 definiert, bei der Herstellung einer pharmazeutischen Zusammensetzung mit Anti-Hypertonie-Wirkung.

**22.** Verwendung nach einem der Ansprüche 7 bis 21, dadurch gekennzeichnet, daß der Zusammensetzung kein Anti-Bakterien- oder Anti-Pilz-Konservierungsmittel und/oder Antioxidans-Konservierungsmittel aufgrund der antibakteriellen oder antioxidativen Wirksamkeit von Oraposid oder seinen Derivaten zugesetzt wird.

**Patentsprüche für folgenden Vertragsstaat : FR**

**1.** Neues Kaffeesäure-Derivat, dadurch gekennzeichnet, daß es sich handelt um Oraposid-Derivate der folgenden Formel (I):

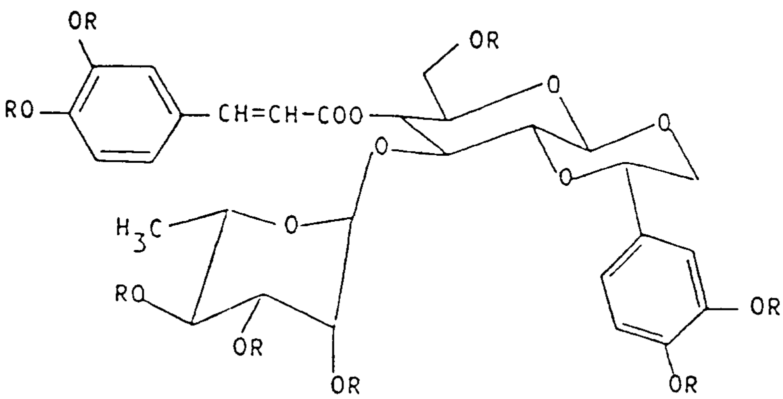

(I),

worin eines oder mehrere R unabhängig ein Wasserstoffatom, eine Alkyl-Gruppe, niederer als $C_1$-$C_5$, insbesondere Methyl, oder eine Acyl-Gruppe, insbesondere $C_1$-$C_6$, spezifisch Acetyl, bedeuten, mit Ausnahme des Falls, in dem alle R gleichzeitig ein Wasserstoffatom darstellen.

**2.** Verbindung nach Anspruch 1, dadurch gekennzeichnet, daß nur die OR-Gruppen, die von den Glucopyranosyl- oder Rhamnopyranosyl-Ringen getragen werden, acyliert, insbesondere acetyliert sind.

**3.** Verbindung nach Anspruch 1 oder 2, dadurch gekennzeichnet, daß nur die OR-Gruppen, die von den Phenyl-Ringen getragen werden, alkyliert, insbesondere methyliert sind.

**4.** Verbindung nach Anspruch 1 bis 3, dadurch gekennzeichnet, daß die Oraposid-Derivate aus einer Pflanze der Familie Orobanchaceae, insbesondere Orobanche rapum genistae, extrahiert werden.

**5.** Kosmetische oder pharmazeutische, insbesondere dermatologische Zusammensetzung, dadurch gekennzeichnet, daß diese als aktiven Bestandteil Oraposid-Derivate der Formel (I), wie in einem der Ansprüche 1 bis 4 definiert, umfaßt.

**6.** Zusammensetzung nach Anspruch 4, dadurch gekennzeichnet, daß diese etwa 0,001 bis etwa 10 Masse-%, vorzugsweise etwa 0,1 bis etwa 5 Masse-%, Oraposid-Derivate, bezogen auf die Gesamtmasse der End-Zusammensetzung, umfaßt.

**7.** Zusammensetzung nach Anspruch 5 oder 6, dadurch gekennzeichnet, daß dieser kein Anti-Bakterien- oder Anti-Pilz-Konservierungsmittel und/oder Antioxidans-Konservierungsmittel aufgrund der antibakteriellen oder antioxidativen Wirksamkeit von Oraposid oder seinen Derivaten zugesetzt wird.

**8.** Verwendung von Oraposid-Derivaten der Formel (I), wie in einem der Ansprüche 1 bis 4 definiert, bei der Herstellung einer kosmetischen oder pharmazeutischen, insbesondere dermatologischen Zusammensetzung mit Wirksamkeit gegen freie Radikale, entzündungshemmender Wirksamkeit, Hepato-Schutzwirksamkeit, Wirksamkeit gegen die Alterung von Zellen, insbesondere gegen die Alterung von

Hautzellen und spezifisch gegen die Alterung durch aktinische Strahlung.

**9.** Verwendung von Oraposid oder seinen Derivaten der folgenden Formel (I):

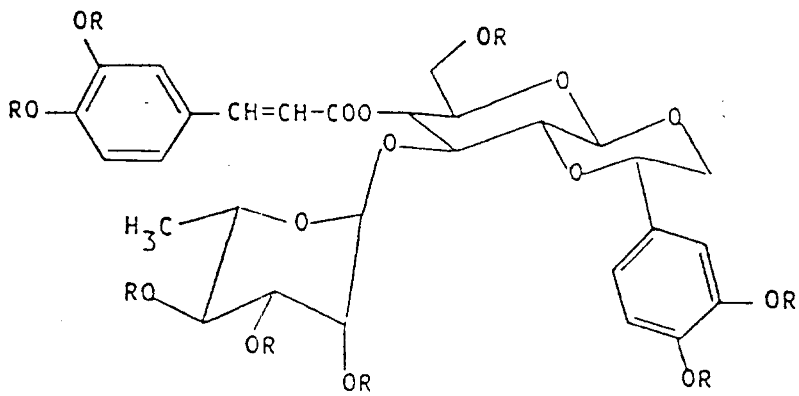

(I),

worin eines oder mehrere R unabhängig ein Wasserstoffatom, eine Alkyl-Gruppe, niederer als $C_1$-$C_5$, insbesondere Methyl, oder eine Acyl-Gruppe, insbesondere $C_1$-$C_6$, spezifisch Acetyl, bedeuten, bei der Herstellung einer kosmetischen oder pharmazeutischen, insbesondere dermatologischen Zusammensetzung mit Wirksamkeit bei der Prävention oder Behandlung von Hautpigmentflecken, Aldosereduktase-Inhibitor-Wirksamkeit, 5-Lipoxygenase-Inhibitor-Wirksamkeit, DOPA-Decarboxylase-Inhibitor-Wirksamkeit, $\beta$-Blocker-Wirksamkeit, Wirksamkeit bei der Filterung von UVA- und UVB-Ultraviolettstrahlung, analgetischer Wirksamkeit, anti-allergischer Wirksamkeit, Anti-Tremor-Wirksamkeit, psychotroper Wirksamkeit und Anti-Hypertonie-Wirkung.

**10.** Verwendung von Oraposid oder seinen Derivaten der Formel (I), wie in Anspruch 9 definiert, bei der Herstellung einer kosmetischen oder pharmazeutischen, insbesondere dermatologischen Zusammensetzung mit Wirksamkeit gegen freie Radikale.

**11.** Verwendung von Oraposid oder seinen Derivaten der Formel (I), wie in Anspruch 9 definiert, bei der Herstellung einer kosmetischen oder pharmazeutischen, insbesondere dermatologischen Zusammensetzung mit entzündungshemmender Wirksamkeit.

**12.** Verwendung von Oraposid oder seinen Derivaten der Formel (I), wie in Anspruch 9 definiert, bei der Herstellung einer pharmazeutischen Zusammensetzung mit Hepato-Schutzwirksamkeit.

**13.** Verwendung von Oraposid oder seinen Derivaten der Formel (I), wie in Anspruch 9 definiert, bei der Herstellung einer kosmetischen oder pharmazeutischen, insbesondere dermatologischen Zusammensetzung mit Wirksamkeit gegen die Alterung von Zellen, insbesondere gegen die Alterung von Hautzellen und spezifisch gegen die Alterung durch aktinische Strahlung.

**14.** Verwendung von Oraposid oder seinen Derivaten der Formel (I), wie in Anspruch 9 definiert, bei der Herstellung einer kosmetischen oder pharmazeutischen, insbesondere dermatologischen Zusammensetzung mit Wirksamkeit bei der Prävention oder Behandlung von Hautpigmentflecken.

**15.** Verwendung von Oraposid oder seinen Derivaten der Formel (I), wie in Anspruch 9 definiert, bei der Herstellung einer pharmazeutischen Zusammensetzung mit Aldosereduktase-Inhibitor-Wirksamkeit.

**16.** Verwendung von Oraposid oder seinen Derivaten der Formel (I), wie in Anspruch 9 definiert, bei der Herstellung einer kosmetischen oder pharmazeutischen, insbesondere dermatologischen Zusammensetzung mit 5-Lipoxygenase-Wirksamkeit.

**17.** Verwendung von Oraposid oder seinen Derivaten der Formel (I), wie in Anspruch 9 definiert, bei der Herstellung einer kosmetischen oder pharmazeutischen, insbesondere dermatologischen Zusammensetzung mit DOPA-Decarboxylase-Inhibitor-Wirksamkeit.

**18.** Verwendung von Oraposid oder seinen Derivaten der Formel (I), wie in Anspruch 9 definiert, bei der Herstellung einer pharmazeutischen Zusammensetzung mit $\beta$-Blocker-Wirksamkeit.

**19.** Verwendung von Oraposid oder seinen Derivaten der Formel (I), wie in Anspruch 9 definiert, bei der Herstellung einer kosmetischen oder pharmazeutischen, insbesondere dermatologischen Zusammensetzung mit Wirksamkeit bei der Filterung von UVA- oder UVB-Ultraviolettstrahlung.

**20.** Verwendung von Oraposid oder seinen Derivaten der Formel (I), wie in Anspruch 9 definiert, bei der Herstellung einer kosmetischen oder pharmazeutischen, insbesondere dermatologischen Zusammensetzung mit analgetischer Wirksamkeit.

**21.** Verwendung von Oraposid oder seinen Derivaten der Formel (I), wie in Anspruch 9 definiert, bei der Herstellung einer kosmetischen oder pharmazeutischen, insbesondere dermatologischen Zusammensetzung mit anti-allergischer Wirksamkeit.

**22.** Verwendung von Oraposid oder seinen Derivaten der Formel (I), wie in Anspruch 9 definiert, bei der Herstellung einer pharmazeutischen Zusammensetzung mit Anti-Tremor-Wirksamkeit.

**23.** Verwendung von Oraposid oder seinen Derivaten der Formel (I), wie in Anspruch 9 definiert, bei der Herstellung einer pharmazeutischen Zusammensetzung mit psychotroper Wirksamkeit.

**24.** Verwendung von Oraposid oder seinen Derivaten der Formel (I), wie in Anspruch 9 definiert, bei der Herstellung einer pharmazeutischen Zusammensetzung mit Anti-Hypertonie-Wirkung.

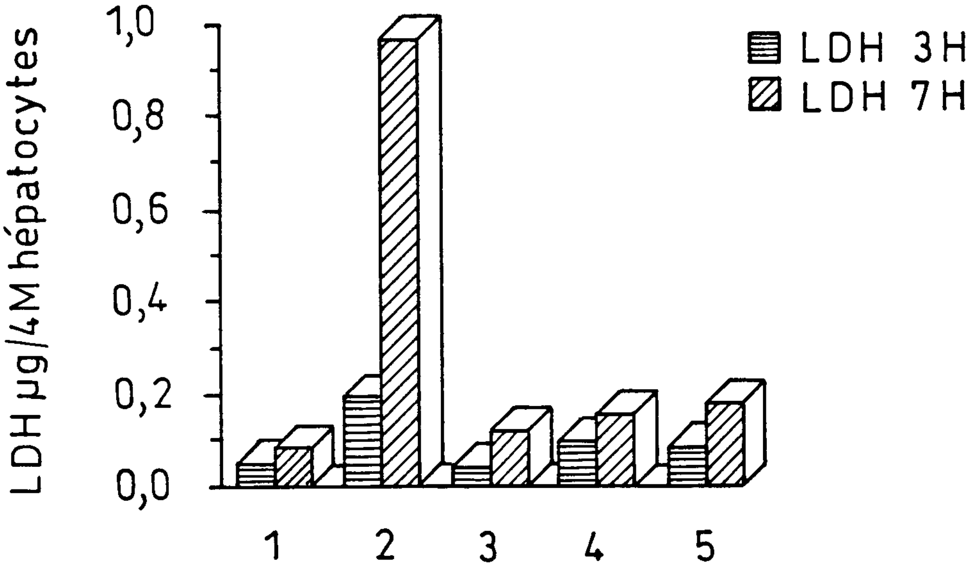
LDH µg/4M hépatocytes
1,0
0,8
0,6
0,4
0,2
0,0
LDH 3H
LDH 7H
1
2
3
4
5

fig_1

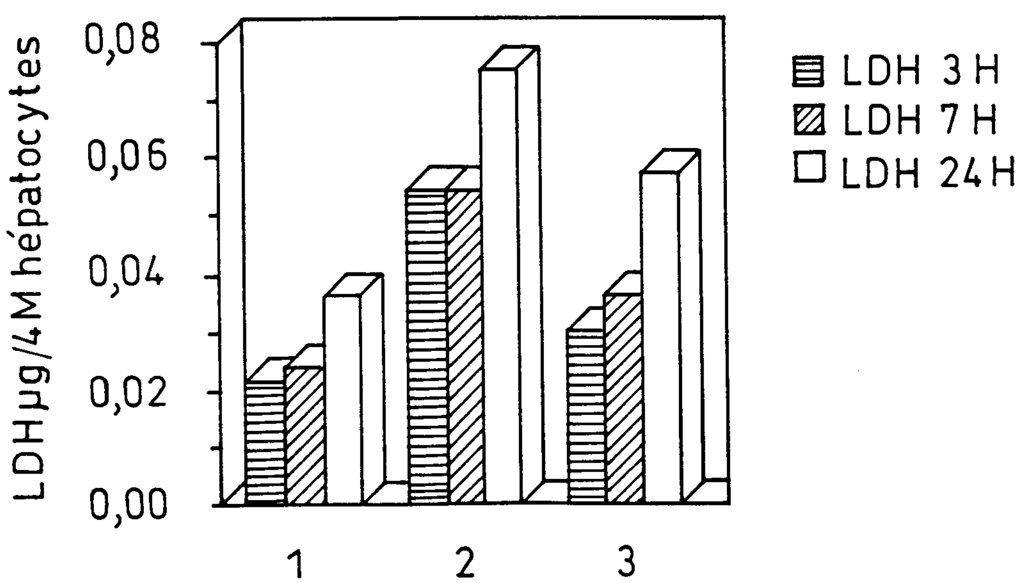
LDHµg/4M hépatocytes
0,08
0,06
0,04
0,02
0,00
LDH 3H
LDH 7H
LDH 24H
1
2
3

fig_2

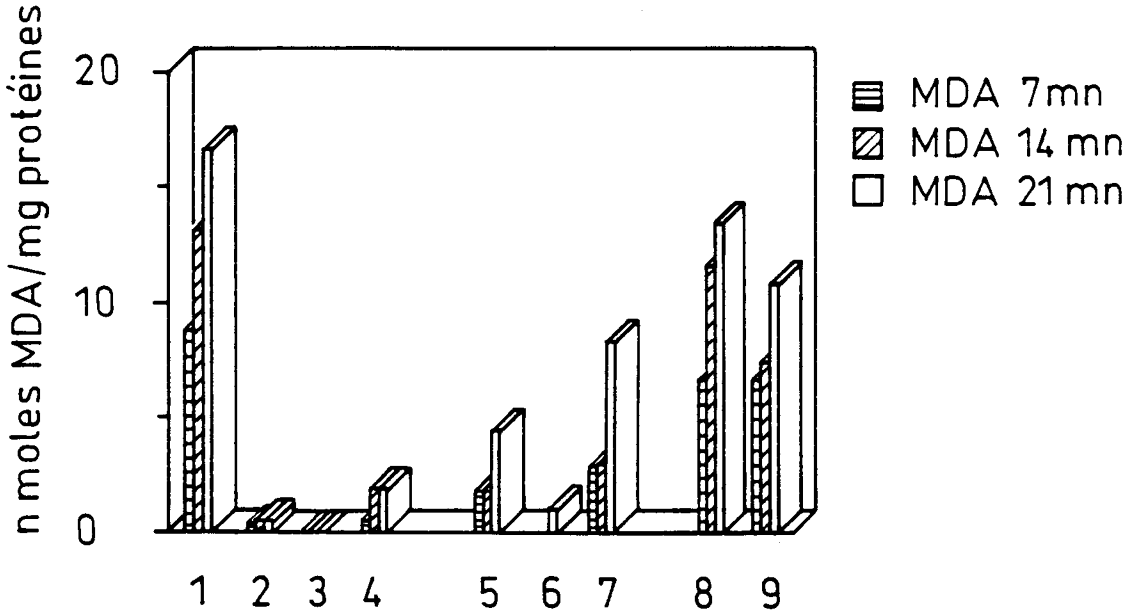
20
10
0
n moles MDA/mg protéines
1 2 3 4 5 6 7 8 9
MDA 7mn
MDA 14 mn
MDA 21 mn

fig_3

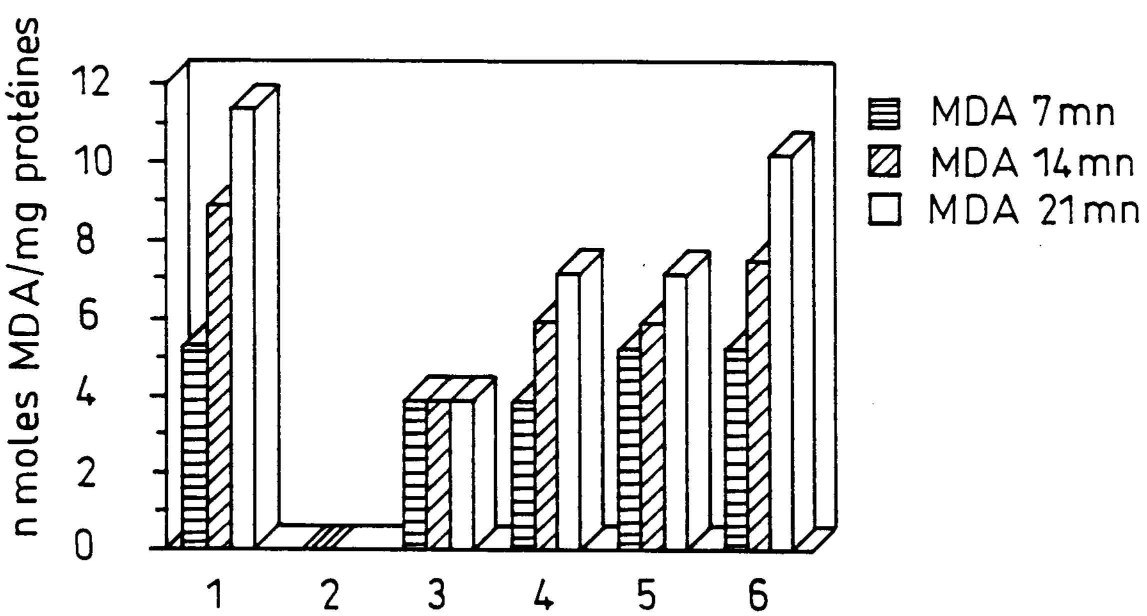
12
10
8
6
4
2
0
n moles MDA/mg protéines
1 2 3 4 5 6
MDA 7mn
MDA 14mn
MDA 21mn

fig_4